# EUROPÄISCHE PATENTSCHRIFT

(11) **EP 0 954 255 B1**
(45) Veröffentlichungstag und Bekanntmachung des Hinweises auf die Patenterteilung: **24.07.2002**
(21) Anmeldenummer: 98905250.1
(22) Anmeldetag: 14.01.1998
(51) Int. Cl.: A61F 2/16

(54) **VERFAHREN ZUR ERMITTLUNG EINER SOLLFORM MINDESTENS EINER VON EINEM STRAHLENGANG DURCH DIE PUPILLE EINES AUGES GESCHNITTENEN OBERFLÄCHE EINES KÜNSTLICHEN ODER NATÜRLICHEN TEILS EINES AUGES UND VORRICHTUNG ZUR HERSTELLUNG EINER KÜNSTLICHEN LINSE**
METHOD FOR DETERMINING THE DESIRED SHAPE OF AT LEAST ONE SURFACE OF A SYNTHETIC OR NATURAL PART OF AN EYE THAT IS INTERSECTED BY A BEAM PATH THROUGH THE EYE PUPIL, AND DEVICE FOR PRODUCTION OF A SYNTHETIC LENS
PROCEDE POUR DETERMINER UNE FORME EXIGEE D'AU MOINS UNE SURFACE D'UNE PARTIE ARTIFICIELLE OU NATURELLE D'UN OEIL, DECOUPEE PAR LE TRAJET D'UN FAISCEAU A TRAVERS LA PUPILLE D'UN OEIL, ET DISPOSITIF POUR PRODUIRE UN CRISTALLIN ARTIFICIEL

(30) Priorität: 21.01.1997 DE 19701807; 26.06.1997 DE 19727121
(43) Veröffentlichungstag der Anmeldung: 10.11.1999
(73) Patentinhaber: TECHNOMED GESELLSCHAFT FÜR MED. UND MED.-TECHN.SYSTEME MBH, 52499 Baesweiler (DE)
(72) Erfinder: VON WALLFELD, Herbert, D-52428 Jülich (DE); NEUHANN, Thomas, D-80803 München (DE)
(74) Vertreter: Castell, Klaus, Dr.
(86) Internationale Anmeldenummer: DE9800102
(87) Internationale Veröffentlichungsnummer: WO9831299

(56) Entgegenhaltungen:
- EP-A- 0 264 255
- EP-A- 0 369 561
- WO-A-92/17134
- WO-A-96/35397
- DE-A- 4 440 573
- US-A- 5 092 880
- US-A- 5 282 852

## Beschreibung

Die Erfindung betrifft ein Verfahren zur Ermittlung einer Sollform mindestens einer von einem Strahlengang durch die Pupille eines Auges geschnittenen Oberfläche eines künstlichen oder natürlichen Teils eines Auges und eine Vorrichtung zur Herstellung einer künstlichen Linse.

Zu den natürlichen Teilen eines Auges, die von einem durch die Pupille eines Auges gehenden Strahlengang geschnitten werden, zählen die Hornhaut und die Linse. Darüber hinaus können auf die Hornhautoberfläche aber auch optische Vorrichtungen aufgelegt oder operativ aufgebracht werden, in das Hornhautinnere können Implatate eingesetzt werden, um die Hornhautform zu variieren, und in den vorderen Augenabschnitt oder in das Augeninnere können weitere Vorrichtungen eingebracht werden, um den durch das Auge gehenden Strahlengang zu brechen.

Die Form der geschnittenen Oberfläche ist von besonderer Bedeutung, da durch die Veränderung der Form einer durch den Strahlengang geschnittenen Oberfläche die Brechkraft des Auges verändert wird. Bei Maßnahmen zur Behandlung von natürlichen Teilen des Auges oder künstlich eingesetzten oder aufgebrachten Teilen sollte der Erfolg genau vorbestimmbar sein.

Insbesondere bei der Abstimmung mehrerer Maßnahmen, die die Brechkraft des Auges verändern, bestehen große Probleme, den tatsächlich erreichten Erfolg vorherzusagen.

Beispielsweise ist zur Herstellung einer künstlichen Linse bekannt, mittels der Keratometrie in 4 Punkten die Krümmung der Augenhornhaut zu vermessen und durch Extrapollation die ungefähre Krümmung der geamten Hornhautoberfläche zu bestimmen. Außerdem werden mit der Biometrie die Abstände zwischen Hornhautvorderfläche, Linse und Retina vermessen, um mittels der durchschnittlichen Hornhautkrümmung die Krümmung bzw. Brechkraft der Linse so festzulegen, daß an der Hornhaut einfallende Strahlen sich in einem Punkt auf der Retina schneiden.

Dieses Verfahren ist für eine Hornhaut mit einer sehr gleichmäßigen Krümmung geeignet. Da die Hornhautkrümmung in der Regel jedoch im Bereich der gesamten Fläche unterschiedlich ist, werden in der Praxis in bestimmten Hornhautbereichen einfallende Strahlen nicht im angestrebten Punkt focusiert.

Außerdem eignet sich dieses Verfahren nur zur Bestimmung der Oberfläche einer künstlichen Linse.

Eine Vorrichtung zur Herstellung einer künstlichen Linse mit einer Einrichtung zum Schneiden der künstlichen Linse nach vorermittelten Werten ist aus der EP-A-0 369561 bekannt. Wie die benötigten Werte ermittelt werden, wird in dieser Veröffentlichung jedoch nicht beschrieben.

Der Erfindung liegt daher die Aufgabe zugrunde, ein Verfahren vorzuschlagen, das es erlaubt, die Sollform einer beliebigen von einem Strahlengang durch die Pupille eines Auges geschnittenen Oberfläche so zu bestimmen, daß eine möglichst genaue Abbildung im Auge entsteht.

Diese Aufgabe wird mit einem Verfahren gelöst, bei dem für eine Vielzahl über die Oberfläche verteilte, jeweils von einem Punkt ausgehende Strahlengänge das Brechungsverhalten längs des Strahlenganges gemessen, die Abstände längs des Strahlengangs der einfallenden Strahlen zwischen den vom Strahlengang geschnittenen Oberflächen gemessen und aus diesen Werten die Sollform mindestens einer von den Strahlengängen geschnittenen Oberfläche so berechnet wird, daß die Strahlengänge sich in einem Punkt schneiden.

Das erfindungsgemäße Verfahren hat den Vorteil, daß auf einer von einem Strahlengang durch die Pupille des Auges geschnittenen Oberfläche beliebig viele Punkte vermessen werden können, um die Genauigkeit für die Formgebung der Oberfläche zu erhöhen. In besonders wichtigen Bereichen, wie z. B. dem zentralen Bereich, können sehr viele Punkte berechnet, während im Randbereich die Berechnung weniger Punkte ausreichen kann. Auch während der Vermessung oder vorher festgestellte Verformungen der Hornhaut oder eines anderen Augenbereiches können bei dem erfindungsgemäßen Verfahren berücksichtigt werden, indem gerade in diesem Bereich besonders viele Punkte vermessen werden. Die mit dem Verfahren bestimmte Sollform ist somit genau an das individuelle Auge angepaßt und kann somit Verformungen oder Unregelmäßigkeiten an einer anderen Stelle des Auges ausgleichen.

Das Verfahren eignet sich vor allem zur Simulation von Eingriffen am Auge. Beispielsweise kann die gesamte Oberfläche einer auf die Augenoberfläche auflegbaren Kontaktlinse berechnet werden. Wenn das notwendige Brechungsverhalten der Kontaktlinse auf zum Kontaktlinsenmittelpunkt konzentrischen Ringen annähernd gleich ist, eignet sich der Einsatz einer Kontaktlinse. Andernfalls muß eine andere Maßnahme vorgenommen werden oder es werden die Abweichungen aufweisende Bereiche der Oberfläche durch ein weiteres Verfahren kompensiert. Dieses Beispiel zeigt, daß das erfindungsgemäße Verfahren einstufig eingesetzt werden kann, um die Sollform einer Oberfläche festzulegen. Da jedoch viele Sollformen technisch oder operativ nicht herstellbar sind, wird in diesen Fällen in einem zweiten Verfahrensschritt unter Berücksichtigung der ersten Maßnahme, wie beispielsweise der aufgelegten Kontaktlinse erneut das Brechungsverhalten längs der Strahlengänge gemessen, um aus den gemessenen Werten die Sollform einer anderen Oberfläche zu bestimmen.

Zu den verschiedenen Eingriffen, für die die genaue Bestimmung der Sollform einer Oberfläche von großer Bedeutung ist, zählen u. a. neben den Kontaktlinsen die Epikeratophakie, die photorefraktive Keratektomie, die radiäre Keratotomie und die Thermokeratoplastik, intrakorneale Inlays, intrakorneale Ringe, die Linsenimplantation in das phake Auge und der Einsatz von Intraokkularlinsen.

Vorteilhaft ist es, wenn zusätzlich zur Ermittlung der Sollform der Oberfläche mit den berechneten Werten der Stoff eines Teiles des Auges nach seinem Brechungsverhalten ausgewählt wird. Während die Dicke des Stoffes, der vom Strahlengang geschnitten wird, bei dem beschriebenen Verfahren in die Sollform der Oberfläche eingeht, bewirkt auch die Auswahl des Materials von künstlich ins Auge eingesetzten Teilen ein spezielles Brechungsverhalten. Das erfindungsgemäße Verfahren erlaubt es somit, die Materialien für die eingesetzten Teile zu variieren und für unterschiedliche Materialien die jeweilige Form der geschnittenen Oberflächen zu bestimmen. Beispielsweise eine künstliche Linse hat je nach verwendetem Material ein anderes Brechungsverhalten und daher wird je nach verwendetem Material eine andere Sollform mit dem erfindungsgemäßen Verfahren bestimmt.

Ein bevorzugter Anwendungbereich des Verfahrens liegt darin, daß mit den berechneten Werten eine Vorrichtung zur Herstellung einer künstlichen Linse angesteuert wird. Die mit dem Verfahren hergestellte Linse ist somit genau an die individuelle Augenhornhaut angepaßt und kann somit Verformungen auf der Hornhaut ausgleichen.

Die Anzahl der vermessenen Punkte, an denen das Brechungsverhalten gemessen wird, ist beliebig zu steigern. Vorzugsweise sollten jedoch mehr als 20 Punkte vermessen werden, um ein akzeptables Ergebnis zu erzielen.

Um eine große Anzahl an Punkten schnell zu vermessen, wird vorgeschlagen, daß das Brechungsverhalten an der Hornhaut topometrisch analysiert wird. Die Topometrie erlaubt eine sehr genaue Vermessung der gesamten Hornhautoberfläche und ist daher für das erfindungsgemäße Verfahren besonders geeignet.

Die Abstände längs des Strahlengangs der einfallenden Strahlen zwischen den vom Strahlengang geschnittenen Oberflächen, wie Hornhaut, Linse und Retina werden vorzugsweise biometrisch gemessen. Dies ist ein einfaches, gut erforschtes und besonders genaues Verfahren für derartige Vermessungen.

Da bekannt ist, daß auch die Dicke der Hornhaut über die Fläche variiert, ist es vorteilhaft, wenn die Hornhautdicke an mehreren Punkten gemessen wird. Üblicherweise wird bei der Herstellung und auch der Implantation künstlicher Linsen die Hornhautdicke nicht speziell berücksichtigt. Die Vermessung der Hornhautdicke führt jedoch zu einer weiteren Steigerung der Genauigkeit, beispielsweise bei der Herstellung künstlicher Linsen.

Je nach Berechnung kann der Schnittpunkt der Strahlengänge vor oder hinter der Retina liegen. Vorteilhaft ist ein Schnittpunkt auf der Retina, vorzugsweise im gelben Fleck.

Im Prinzip reicht es aus, eine Oberfläche genauestens zu berechnen und entsprechend zu verformen, um einen einzigen Schnittpunkt aller Strahlengänge zu erzielen. Gerade bei notwendigen stärkeren Verformungen oder aus herstellungstechnischen oder operativen Gründen ist es jedoch oftmals günstig, wenn die Sollformen mehrere Oberflächen berechnet werden. Beispielsweise kann eine Linsenvorderseite und die Linsenrückseite einer künstlichen Linse individuell geformt werden, so daß das gesamte Brechungsverhalten der Linse zusammen mit dem Brechungsverhalten der übrigen Oberflächen des Auges zu dem erfindungsgemäßen Schnittpunkt führen.

Für Menschen, die wegen der Alterssichtigkeit für Ferne und Nähe verschiedene Brillengläser benötigen, empfiehlt es sich, daß die Brechkraft der Oberflächen so berechnet wird, daß ein Gleitsicht, Bi- oder Multifokaleffekt entsteht. Dies kann beispielsweise durch spezielle Krümmungen einer künstlich eingesetzten Linse erreicht werden.

Außerdem liegt der Erfindung die Aufgabe zugrunde, eine Vorrichtung zur Herstellung einer künstlichen Linse vorzuschlagen, die es erlaubt, Unebenheiten auf der Hornhaut zu berücksichtigen.

Diese Aufgabe wird gelöst mit einer Vorrichtung zur Herstellung einer künstlichen Linse mit einer Eingabeeinheit zum Eingeben der Hornhautkrümmung und des Ortes einer Vielzahl von Punkten auf der Augenhornhaut und der Abstände zwischen Hornhaut, einzusetzender Linse und Retina, mit einem Rechner zur Berechnung des Brechungswinkels für jeden dieser Punkte und der Krümmung jedes einem Punkt auf der Hornhaut entsprechenden Punktes auf der Linsenoberfläche und einer Einrichtung zur Herstellung der künstlichen Linse nach den berechneten Werten.

Eine derartige Vorrichtung erlaubt es, auf der Grundlage der notwendigen, beispielsweise vom Optiker oder Augenarzt ermittelten Daten eines speziellen Auges, eine individuelle Linse herzustellen, die Unebenheiten auf der Hornhaut ausgleicht. Die gemessenen Werte können beispielsweise auf einer Diskette oder online dem Linsenhersteller übermittelt werden, der eine den Werten entsprechende Linse herstellt.

Vorzugsweise weist die Einrichtung zum Herstellen der künstlichen Linse eine Lasereinrichtung zum Schneiden der Linse auf. Lasereinrichtungen erlauben eine genaue Steuerung von Schnitten und sind somit besonders geeignet, auf der Grundlage von vorgegebenen Daten automatisch eine entsprechende Linse zu schneiden.

Die so erzeugte Linse kann durch Mittelwertbildung der Werte auf konzentrischen Ringen um den Mittelpunkt eine auf jedem Radius gleiche Form erhalten. Eine höhere Genauigkeit wird jedoch erreicht, wenn die Linse nicht konzentrisch, sondern möglichst genau der Hornhaut entsprechend ausgebildet ist.

In diesem Fall muß die erzeugte Linse mittels Hilfsinstrumenten oder Aufhängevorrichtungen in einer genau festgelegten Ausrichtung in das menschliche Auge eingesetzt werden, da schon eine kleine Verdrehung der Linse die genaue Anpassung an die Augenhornhaut zunichte macht. Es wird daher vorgeschlagen, die hergestellte Linse mit einer Markierung zu versehen, die beim Einsetzen der Linse die Ausrichtung der Linse erleichtert.

Das erfindungsgemäße Verfahren und ein Ausführungsbeispiel zur erfindungsgemäßen Vorrichtung werden in den Zeichnungen dargestellt und im folgenden näher beschrieben.

Es zeigt,
- Figur 1: einen waagerechten, schematischen Schnitt durch den rechten Augapfel, von oben gesehen.
- Figur 2: eine schematische Darstellung der Vorrichtung.

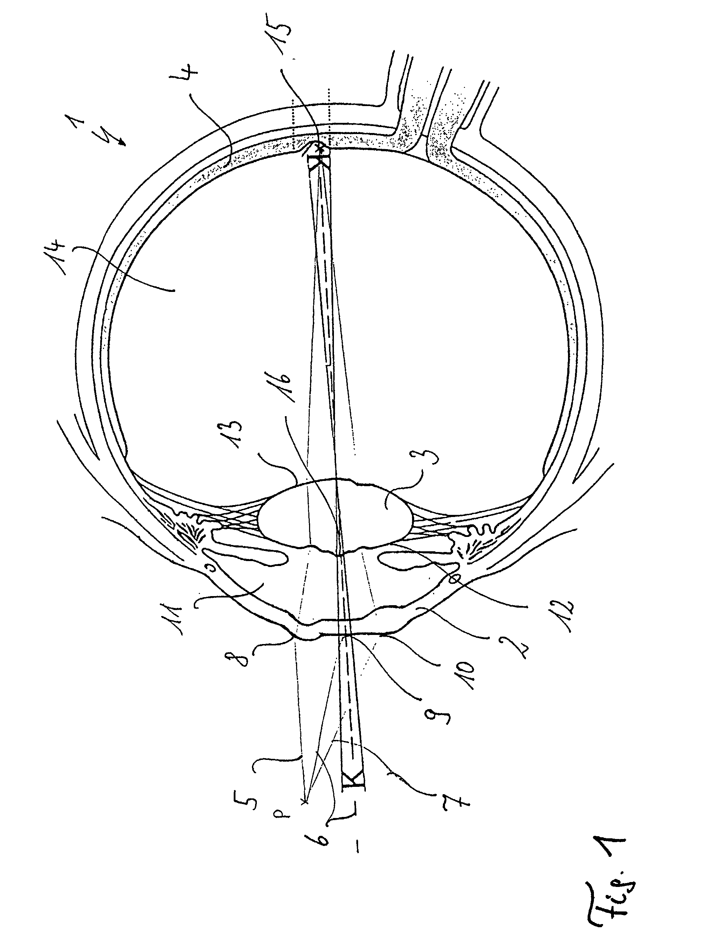

Das in Figur 1 gezeigte Auge 1 besteht im wesentlichen aus der Hornhaut 2, der künstlichen Linse 3 und der Retina 4. Von einem Punkt P ausgehende Strahlen 5, 6, 7 treffen an den Punkten 8, 9, 10 auf die Hornhaut 2, gehen durch die Hornhaut hindurch in die vordere Augenkammer 11, treffen auf die Linsenvorderseite 12, gehen durch die künstliche Linse 3 hindurch und gelangen über die Linsenrückseite 13 in den Glaskörper 14. Im weiteren Verlauf durch den Glaskörper sind die aus der künstlichen Linse heraustretenden Strahlen 5, 6, 7, so abgelenkt, daß sie sich in einem gemeinsamen Schnittpunkt 15 treffen.

Bei einer sphärisch gekrümmten Hornhaut 2 kann an einem oder wenigen Punkten die Hornhautkrümmung beispielsweise keratometrisch gemessen werden, um die Linsenkrümmung der künstlichen Linse 3 so zu bestimmen, daß die einfallenden Strahlen 5, 6, 7, sich in idealer Weise einem Schnittpunkt in diesem Beispiel dem gelben Fleck 15 treffen.

Problematisch ist jedoch, wenn die Hornhaut 2 wie im Punkt 8, eine abweichende Krümmung aufweist oder wie im Punkt 10, eine erhöhte Dicke. An diesen Stellen ist die Hornhaut 2 nicht vollständig sphärisch und die einfallenden Strahlen werden so gebrochen, daß sie nach dem Durchgang durch die künstliche Linse sich nicht mehr im Punkt 15 treffen. Daher wird nach der Erfindung vorgeschlagen, daß die Linsenoberfläche 12, 13 so gestaltet wird, daß Abweichungen in der Hornhautkrümmung oder der Hornhautdicke durch die Oberflächengestaltung an der künstlichen Linse ausgeglichen werden. Hierbei kann entweder nur eine Seite der Linse der Hornhaut entsprechend bearbeitet werden oder es können die Vorderseite 12 und die Rückseite 13 wie am Strahlengang 7 gezeigt, derart variiert werden, daß durch kleine Veränderungen der Krümmungen an der künstlichen Linse 3 Besonderheiten im Bereich der Hornhaut 2 ausgeglichen werden. Beispielsweise kann auch mit einer Seite der Linse 3 die Krümmung und mit der anderen Seite der Linse 3 die Dicke der Hornhaut ausgeglichen werden.

Zur Berechnung der Krümmung jedes einem Punkt 8, 9, 10 auf der Hornhaut 2 entsprechenden Punktes auf der Linsenoberfläche 12, 13 ist es notwendig, die Abstände längs des Strahlenganges einfallender Strahlen 5, 6, 7 zwischen Hornhaut 2, einzusetzender Linse 3 und Retina 4 zu vermessen. Diese Daten können mittels der Biometrie ermittelt werden, die auf eine Genauigkeit von einem Zehntel Millimeter die Abstände zwischen Hornhaut 2, Vorderseite 12 der künstlichen Linse 3, Rückseite 13 der künstlichen Linse 3 und der Retina angibt.

Wenn diese Daten bekannt sind, kann mittels der Topometrie über eine Vielzahl an Punkten 8, 9, 10 die genaue Krümmung der Hornhaut 2 über die gesamte Fläche der Hornhaut 2 ermittelt werden, um daraus den Brechungswinkel in jedem der Punkte 8, 9, 10 zu bestimmen. Die Genauigkeit der Messung wird noch dadurch erhöht, daß in jedem der Punkte 8, 9, 10 zusätzlich die Hornhautdicke bestimmt wird.

Mit den derart berechneten Werten wird eine Vorrichtung 20 zur Herstellung einer künstlichen Linse angesteuert, die aus den gemessenen und berechneten Werten eine künstliche Linse erstellt.

Im Ausführungsbeispiel werden auf der Hornhaut 3 topometrisch mehrere tausend Punkte gemessen und dazu die relevanten Werte in einem Modell berechnet. Der Schnittpunkt liegt auf der Retina, vorzugsweise im gelben Fleck. Verständlicherweise kann die Krümmung der Linsenoberfläche im Einzelfall aber auch so berechnet werden, daß der Schnittpunkt 15 auf einem anderen Ort auf der Retina oder vor bzw. hinter der Retina liegt.

Mit dem beschriebenen Verfahren lassen sich Fehlsichtigkeiten, die auf eine nicht exakt sphärische, asphärische oder irreguläre Krümmung der einfallenden Strahlen oder auf Kurzbau oder Langbau des Auges (Achsenametropie) zurückzuführen sind, begleichen. Eine Fehlsichtigkeit in Folge einer ungenügenden Anpassung der künstlichen Linse an unterschiedliche Sichtweiten ist im vorliegendem Beispiel durch eine überproportionale Krümmung der künstlichen Linse 3 in ihrem zentralen Bereich 16 auf ihrer Vorderseite 12 ausgeglichen.

Die Verformungen auf der Hornhaut 2 und den Oberflächen 12, 13 der künstlichen Linse 3 sind überproportional vergrößert eingezeichnet, um sichtbar zu sein.

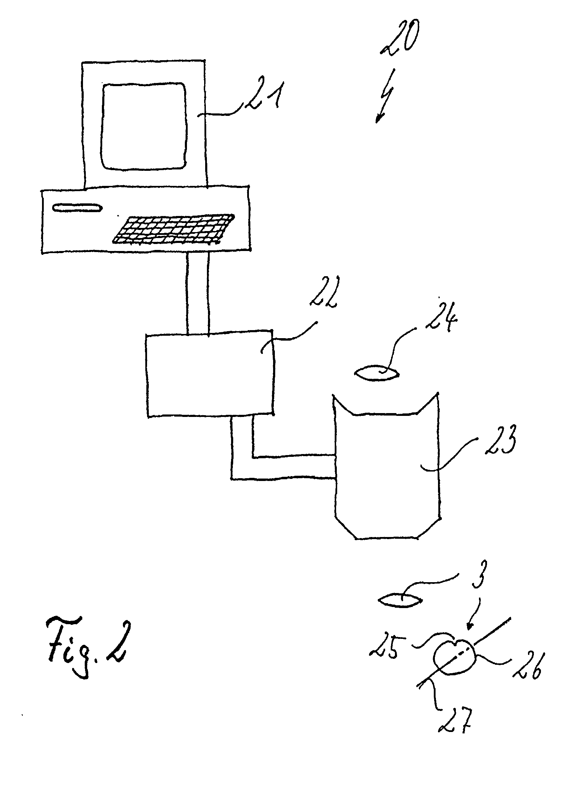

Figur 2 zeigt schematisch eine Vorrichtung 20 zum Schneiden einer künstlichen Linse 3. Diese Vorrichtung 20 besteht aus einer Eingabeeinheit 21 zum Eingeben der Hornhautkrümmung und des Ortes einer Vielzahl von Punkten 8, 9, 10 auf der Augenhornhaut 2 sowie der Abstände zwischen der Hornhaut 2, der einzusetzenden Linse 3 und der Retina 4. Diese Eingabeeinheit ist für eine mechanische Eingabe ausgebildet. Zusätzlich ist jedoch auch eine Eingabe online oder über zuvor beschriftete Datenträger möglich, die es erlaubt in kürzester Zeit die große Anzahl der benötigten Daten in die Vorrichtung 20 einzugeben.

Der an die Eingabeeinheit 21 angeschlossene Rechner 22 dient dazu, aus vorgegebenen fixen Werten und den eingegebenen Werten, die Brechungswinkel für jeden dieser Punkte 8, 9, 10 zu bestimmen und daraus die Krümmung jedes einem Punkt auf der Hornhaut 2 entsprechenden Punktes auf der Linsenoberfläche 12, 13 zu berechnen.

Diese berechneten Werte werden nun einer Vorrichtung 23 zum Schneiden der künstlichen Linse 3 aus einem Rohling 24 zugeführt, die mittels Lasereinrichtungen aus dem Rohling 24 die für einen individuellen Einsatz berechnete Linse 3 schneidet. Eine Markierung 25 wird durch eine kleine Kerbe am Umfangsrand 26 der Linse 3 erzeugt. Diese Kerbe beeinträchtigt die Funktion der Linse 3 nicht. Sie zeigt aber dem Arzt, der die Linse einsetzt, wie die um ihre zentrale Achse 27 drehbare Linse auszurichten ist.

## Patentansprüche

1. Verfahren zur Ermittlung einer Sollform mindestens einer von einem Strahlengang (5, 6, 7) durch die Pupille eines Auges geschnittenen Oberfläche (12, 13) eines künstlichen (3) oder natürlichen Teiles eines Auges, bei dem
- für eine Vielzahl über die Oberfläche (12, 13) verteilte jeweils von einem Punkt (8) ausgehende Strahlengänge (5, 6, 7) das Brechungsverhalten längs des Strahlenganges (5, 6, 7) gemessen wird,
- die Abstände längs des Strahlenganges der einfallenden Strahlen zwischen den vom Strahlengang geschnittenen Oberflächen (12, 13) gemessen oder aus Erfahrungswerten vorgegeben werden und
- aus diesen Werten die Sollform mindestens einer von den Strahlengängen geschnittenen Oberfläche (12, 13) so berechnet wird, daß die Strahlengänge (5, 6, 7) sich in einem Punkt (15) schneiden.

2. Verfahren nach Anspruch 1, ***dadurch gekennzeichnet, daß*** mit den berechneten Werten das Material von künstlich ins Auge eingesetzten Teilen (3) nach seinem Brechungsverhalten ausgewählt wird.

3. Verfahren nach einem der vorhergehenden Ansprüche, ***dadurch gekennzeichnet, daß*** mit den berechneten Werten eine Vorrichtung (20) zur Herstellung einer künstlichen Linse (3) angesteuert wird.

4. Verfahren nach einem der vorhergehenden Ansprüche, ***dadurch gekennzeichnet, daß*** das Brechungsverhalten an mehr als 20 Punkten (8, 9, 10) gemessen wird.

5. Verfahren nach einem der vorhergehenden Ansprüche, ***dadurch gekennzeichnet, daß*** das Brechungsverhalten an der Hornhaut *(2)* topometrisch analysiert wird.

6. Verfahren nach einem der vorhergehenden Ansprüche, ***dadurch gekennzeichnet, daß*** die Abstände biometrisch gemessen werden.

7. Verfahren nach einem der vorhergehenden Ansprüche, ***dadurch gekennzeichnet, daß*** die Hornhautdicke an mehreren Punkten (8, 9, 10) gemessen wird.

8. Verfahren nach einem der vorhergehenden Ansprüche, ***dadurch gekennzeichnet, daß*** der Schnittpunkt (15) auf der Retina (4), vorzugsweise im gelben Fleck, liegt.

9. Verfahren nach einem der vorhergehenden Ansprüche, ***dadurch gekennzeichnet, daß*** die Sollformen mehrerer Oberflächen (12, 13) berechnet werden.

10. Verfahren nach einem der vorhergehenden Ansprüche, ***dadurch gekennzeichnet, daß*** die Brechungskraft der Oberflächen (12, 13) so berechnet wird, daß ein Gleitsicht, Bi- oder Multifokaleffekt entsteht.

11. Vorrichtung (20) zur Herstellung einer künstlichen Linse (3)
- mit einer Einrichtung (23) zum Schneiden der künstlichen Linse (3) gemäß vorher berechneter Werte, ***gekennzeichnet durch*** eine Eingabeeinheit (21) zum Eingeben der Hornhautkrümmung und des Ortes einer Vielzahl von Punkten (8, 9, 10) auf der Hornhaut (2) und der Abstände zwischen Hornhaut (2), einzusetzender Linse (3) und Retina (4)
- und einen Rechner (22) zur Berechnung des Brechungswinkels für jeden dieser Punkte (8, 9, 10) und der Krümmung jedes einem Punkt (8, 9, 10) auf der Hornhaut (2) entsprechenden Punktes auf der Linsenoberfläche (12, 13) als Ausgangswert für die Einrichtung (23) zum Schneiden der künstlichen Linse (3).

12. Vorrichtung nach Anspruch 11, ***dadurch gekennzeichnet, daß*** die Einrichtung zum Schneiden der künstlichen Linse (3) eine Lasereinrichtung aufweist.

## Claims

1. A method of determining the ideal shape of at least one surface (12, 13) of an artificial part (3) or a natural part of an eye, said surface being intersected by the path of a beam (5, 6, 7) through the pupil of the eye, whereby
- for a plurality of beam paths (5, 6, 7) distributed over the surface (12, 13), each emanating from a point (8), the refraction behavior along the beam path (5, 6, 7) is measured,
- the distances along the beam path of the incident beams between the surfaces (12, 13) intersected by the beam path are measured or predetermined from empirical values, and
- from these values, the ideal shape of at least one surface (12, 13) intersected by the beam paths is calculated so that the beam paths (5, 6, 7) intersect at a point (15).

2. The method according to Claim 1, **characterized in that** the material of parts (3) artificially inserted into the eye is selected according to its refraction behavior by using these calculated values.

3. The method according to one of the preceding claims, **characterized in that** a device (20) for producing an artificial lens (3) is controlled by using the calculated values.

4. The method according to one of the preceding claims, **characterized in that** the refraction behavior is measured at more than 20 points (8, 9, 10).

5. The method according to one of the preceding claims, **characterized in that** the refraction behavior is analyzed topometrically on the cornea.

6. The method according to one of the preceding claims, **characterized in that** the distances are measured biometrically.

7. The method according to one of the preceding claims, **characterized in that** the thickness of the cornea is measured at several points (8, 9, 10).

8. The method according to one of the preceding claims, **characterized in that** the point of intersection (15) is located on the retina (4), preferably in the yellow spot.

9. The method according to one of the preceding claims, **characterized in that** the ideal shapes of several surfaces (12, 13) are calculated.

10. The method according to one of the preceding claims, **characterized in that** the refractive power of the surfaces (12, 13) is calculated so as to yield a progressive lens effect, a bifocal effect or a multifocal effect.

11. A device (20) for producing an artificial lens (3)
- having a device (23) for cutting the artificial lens (3) according to previously calculated values, **characterized by** an input unit (21) for input of the curvature of the cornea and the location of a plurality of points (8, 9, 10) on the cornea (2) and the distances between the cornea (2), the lens (3) to be inserted and the retina (4),
- and a computer (22) for calculating the angle of refraction for each of these points (8, 9, 10) and the curvature of each point on the surface (12, 13) of the lens corresponding to a point (8, 9, 10) on the cornea (2) as the starting value for the device (23) for cutting the artificial lens (3).

12. The device according to Claim 11, **characterized in that** the device for cutting the artificial lens (3) has a laser apparatus.

## Revendications

1. Procédé pour déterminer une forme théorique d'au moins une surface (12, 13) d'une partie artificielle (3) ou naturelle d'un oeil, surface qui est découpée par le trajet d'un faisceau (5, 6, 7) à travers la pupille d'un oeil,
- où pour un grand nombre de trajets de rayon (5, 6, 7) répartis sur la surface (12, 13) et partant respectivement d'un point (8), la force de réfraction est mesurée le long du trajet du rayon (5, 6, 7),
- les écarts le long du trajet de rayon des rayons incidents entre les surfaces (12, 13) coupées par le trajet de rayon sont mesurés ou prédéterminés à partir de valeurs expérimentales et
- à partir de ces valeurs, la forme théorique d'au moins une surface (12, 13) coupées par les trajets de rayon est calculée de sorte que les trajets de rayon (5, 6, 7) se coupent en un point (15).

2. Procédé selon la revendication 1, **caractérisé en ce que** le matériau de parties (3) insérées artificiellement dans l'oeil est sélectionné avec les valeurs calculées selon son indice de réfraction.

3. Procédé selon l'une des revendications précédentes, **caractérisé en ce qu'**un dispositif (20) destiné à la fabrication d'un cristallin artificiel (3) est piloté avec les valeurs calculées.

4. Procédé selon l'une des revendications précédentes, **caractérisé en ce que** la force de réfraction est mesurée sur plus de 20 points (8, 9, 10).

5. Procédé selon l'une des revendications précédentes, **caractérisé en ce que** la force de réfraction est analysée sur la cornée par topométrie.

6. Procédé selon l'une des revendications précédentes, **caractérisé en ce que** les distances sont mesurées biométriquement.

7. Procédé selon l'une des revendications précédentes, **caractérisé en ce que** l'épaisseur de la cornée est mesurée en plusieurs points (8, 9, 10).

8. Procédé selon l'une des revendications précédentes, **caractérisé en ce que** le point d'intersection (15) se trouve sur la rétine (4), de préférence dans la tâche jaune.

9. Procédé selon l'une des revendications précédentes, **caractérisé en ce que** les formes théoriques de plusieurs surfaces (12, 13) sont calculées.

10. Procédé selon l'une des revendications précédentes, **caractérisé en ce que** la puissance de réfraction des surfaces (12, 13) est calculée de sorte qu'il se produit un effet progressif, bi- ou multifocal.

11. Dispositif (20) pour la fabrication d'un cristallin artificiel (3) comprenant
- un dispositif (23) pour découper le cristallin artificiel (3) d'après les valeurs calculées auparavant, **caractérisé par** une unité d'entrée (21) pour la saisie de la courbure de la cornée et de l'emplacement d'un grand nombre de points (8, 9, 10) sur la cornée (2) et les distances entre la cornée (2), le cristallin à placer (3) et la rétine (4)
- et un calculateur (22) pour le calcul de l'angle de réfraction pour chacun de ces points (8, 9, 10) et de la courbure de chaque point correspondant à un point (8, 9, 10) sur la cornée (2), sur la surface du cristallin (12, 13), comme valeur initiale pour le dispositif (23) à découper le cristallin artificiel (3).

12. Dispositif selon la revendication 11, **caractérisé en ce que** le dispositif à découper le cristallin artificiel (3) présente un dispositif à laser.
